# EUROPEAN PATENT APPLICATION

(11) **EP 4 401 042 A1**
(43) Date of publication of application: **17.07.2024**
(21) Application number: 22867582.3
(22) Date of filing: 16.08.2022
(51) Int. Cl.: G06T 17/20, G06T 7/73, A61C 9/00, G06T 7/11, G16H 30/40

(54) **ELECTRONIC DEVICE AND SCANNED IMAGE PROCESSING METHOD OF THREE-DIMENSIONAL SCANNER THEREOF**

(30) Priority: 10.09.2021 KR 20210120791
(71) Applicant: Medit Corp., Seoul 07207 (KR)
(72) Inventor: LEE, Dong Hoon, Seoul 07207 (KR); KANG, Dong Hwa, Seoul 07207 (KR)
(74) Representative: MFG Patentanwälte Meyer-Wildhagen Meggle-Freund Gerhard PartG mbB
(86) International application number: PCT/KR2022/012175
(87) International publication number: WO 2023/038313

(57) **Abstract**

According to various embodiments of the present disclosure, an electronic device is configured to obtain scan data values for a surface of a target object through a scan of a three-dimensional scanner, the scan data values including a three-dimensional coordinate value, generate a three-dimensional image model of the target object based on the obtained scan data values, divide the three-dimensional image model into multiple clusters, determine at least one cluster having a size equal to or smaller than a predetermined size among the multiple clusters, and remove scan data values associated with the at least one cluster.

## Description

### TECHNICAL FIELD

The present disclosure relates to an electronic device and a method of processing a scan image of a three-dimensional scanner thereof. Specifically, the present disclosure relates to a method and an electronic device for locally filtering out noise existing in a three-dimensional image model generated based on an image obtained by scanning of a three-dimensional scanner.

### BACKGROUND

A three-dimensional intraoral scanner is an optical device that is inserted into a patient's oral cavity to scan teeth so as to obtain a three-dimensional image of the oral cavity. By scanning a patient's oral cavity by means of such a three-dimensional scanner, multiple two-dimensional images of the patient's oral cavity may be obtained, and a three-dimensional image of the patient's oral cavity may be constructed using the obtained multiple two-dimensional images. For example, a doctor may inert a three-dimensional scanner into a patient's oral cavity to scan the patient's teeth, gums, and/or soft tissues, thereby obtaining multiple two-dimensional images of the patient's oral cavity. Thereafter, by applying a three-dimensional modeling technology, a three-dimensional image of the patient's oral cavity may be constructed using the two-dimensional images of the patient's oral cavity.

### SUMMARY

If an object other than a target object to be scanned is interposed during the above scanning operation, for example, if a user's finger or other treatment instruments are interposed between a three-dimensional scanner and a tooth during a tooth scanning operation, a tooth part hidden by the interposed object is not scanned and the interposed object may be scanned instead. In this case, a noise image caused by the interposed object may be generated in a constructed three-dimensional image model. If noise occurs, the acquisition of a precise three-dimensional image model of a desired target object becomes impossible, and thus it is required to effectively remove such noise in constructing a three-dimensional image model.

Furthermore, even when such noise is removed using various filtering methods, some noise may not be removed cleanly and thus makes it impossible to obtain a precise three-dimensional image model. Therefore, it is necessary to remove all noise cleanly.

In addition, noise may occur in a process of editing (e.g., removing) some of scan data, and this also prevents acquisition of a precise three-dimensional image model. Therefore, it is necessary to effectively remove such noise.

According to various embodiments of the present disclosure, noise which may exist in a three-dimensional image model generated using a three-dimensional scanner can be effectively removed.

An electronic device comprising: a communication circuit communicatively connected to a three-dimensional scanner; a display; and one or more processors. The one or more processors are configured to: obtain scan data values for a surface of a target object through a scan of the three-dimensional scanner, the scan data values including a three-dimensional coordinate value; generate a three-dimensional image model of the target object, based on the obtained scan data values; divide the three-dimensional image model into multiple clusters; determine at least one cluster having a size equal to or smaller than a predetermined size among the multiple clusters; and remove scan data values associated with the at least one cluster.

A method of processing a scan image of a three-dimensional scanner performed in an electronic device, the method comprising: obtaining scan data values for a surface of a target object through a scan of the three-dimensional scanner, the scan data values including a three-dimensional coordinate value; generating a three-dimensional image model of the target object, based on the obtained scan data values; dividing the three-dimensional image model into multiple clusters; determining at least one cluster having a size equal to or smaller than a predetermined size among the multiple clusters; and removing scan data values associated with the at least one cluster.

According to various embodiments of the present disclosure, the accuracy of a three-dimensional image model of a desired target object can be improved by removing noise existing in scan data values.

According to various embodiments of the present disclosure, noise included in a three-dimensional image model can be effectively removed by dividing the three-dimensional image model into multiple clusters and removing at least one cluster determined as the noise.

### BRIEF DESCRIPTION OF DRAWINGS

FIG. 1 is a diagram illustrating obtaining an image of a patient's oral cavity by means of an oral cavity scanner according to various embodiments of the present disclosure.
FIG. 2A is a block diagram of an electronic device and an oral cavity scanner according to various embodiments of the present disclosure, and FIG. 2B is a perspective view of an oral cavity scanner according to various embodiments of the present disclosure.
FIG. 3 is a diagram illustrating a method of generating a three-dimensional image 320 of an oral cavity according to various embodiments.
FIG. 4A and FIG. 4B are diagrams illustrating a process of performing noise filtering according to various embodiments of the present disclosure.
FIG. 5 is an operation flowchart of an electronic device according to various embodiments of the present disclosure.
FIG. 6 illustrates an interface for generating a three-dimensional image model of a target object according to various embodiments of the present disclosure.
FIG. 7 is an operation flowchart of an electronic device according to various embodiments of the present disclosure.

### DETAILED DESCRIPTION

Embodiments of the present disclosure are illustrated for describing the technical idea of the present disclosure. The scope of the claims according to the present disclosure is not limited to the embodiments described below or to the detailed descriptions of these embodiments.

All technical or scientific terms used in the present disclosure have meanings that are generally understood by a person having ordinary knowledge in the art to which the present disclosure pertains, unless otherwise specified. The terms used in the present disclosure are selected for the purpose of clearer explanation of the present disclosure, and are not intended to limit the scope of claims according to the present disclosure.

The expressions "include," "provided with," "have" and the like used in the present disclosure should be understood as open-ended terms connoting the possibility of inclusion of other embodiments, unless otherwise mentioned in a phrase or sentence including the expressions.

A singular expression used in the present disclosure can include meanings of plurality, unless otherwise mentioned, and the same is applied to a singular expression recited in the claims. The terms "first," "second," etc. used in the present disclosure are used to distinguish a plurality of elements from one another, and are not intended to limit the order or importance of the relevant elements.

The term "unit" used in the present disclosure means a software element or hardware element, such as a field-programmable gate array (FPGA) or an application specific integrated circuit (ASIC). However, a "unit" is not limited to software and hardware. A "unit" may be configured to be stored in an addressable storage medium or may be configured to run on one or more processors. Therefore, for example, a "unit" may include elements, such as software elements, object-oriented software elements, class elements, and task elements, as well as processors, functions, attributes, procedures, subroutines, segments of program codes, drivers, firmware, micro-codes, circuits, data, databases, data structures, tables, arrays, and variables. Functions provided in elements and "unit" may be combined into a smaller number of elements and "units" or further subdivided into additional elements and "units."

The expression "based on" used in the present disclosure is used to describe one or more factors that influences a decision, an action of determination, or an operation described in a phrase or sentence including the relevant expression, and this expression does not exclude an additional factor influencing the decision, the action of determination, or the operation.

In the present disclosure, when a certain element is described as being "coupled to" or "connected to" another element, it should be understood that the certain element may be connected or coupled directly to the other element or that the certain element may be connected or coupled to the other element via a new intervening element.

Hereinafter, embodiments of the present disclosure will be described with reference to the accompanying drawings. In the accompanying drawings, identical or corresponding elements are indicated by identical reference numerals. In the following description of embodiments, repeated descriptions of the identical or corresponding elements will be omitted. However, even when a description of an element is omitted, such an element is not intended to be excluded in an embodiment.

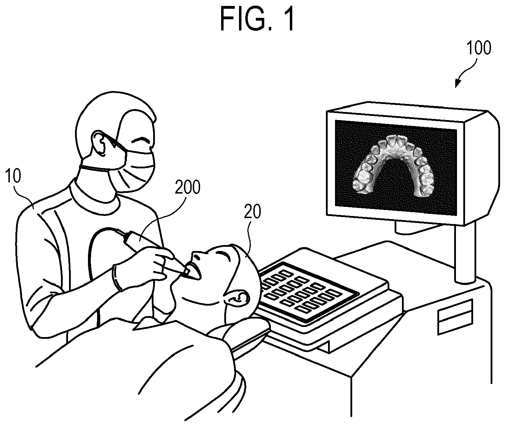

FIG. 1 is a diagram illustrating obtaining an image of a patient's oral cavity by means of a three-dimensional scanner 200 according to various embodiments of the present disclosure. According to various embodiments, the three-dimensional scanner 200 may be a dental medical device for obtaining an image in the oral cavity of a target object 20. For example, the three-dimensional scanner 200 may be an intraoral scanner. As illustrated in FIG. 1, a user 10 (e.g., a dentist or a dental hygienist) may obtain an image of the oral cavity of the target object 20 (e.g., patient) from the target object 20 by using the three-dimensional scanner 200. As another example, the user 10 may obtain an image of the oral cavity of the target object 20 from a diagnostic model (e.g., a plaster model or an impression model) obtained by taking an impression of the shape of the oral cavity of the target object 20. Hereinafter, for convenience of explanation, an image of the oral cavity of the target object 20 being obtained by scanning the oral cavity of the target object 20 is described. However, the disclosure is not limited thereto, and obtaining an image of a different portion (e.g., ears of the target object 20) of the target object 20 is also possible. The three-dimensional scanner 200 may have a shape capable of being introduced into and discharged from an oral cavity, and may be a handheld scanner for which a scan distance and a scan angle are freely adjustable by the user 10.

The three-dimensional scanner 200 according to various embodiments may obtain an image of the oral cavity of the target object 20 by being inserted into the oral cavity and scanning the inside of the oral cavity in a non-contact manner. The image of the oral cavity may include at least one tooth, a gum, and an artificial structure insertable in the oral cavity (e.g., orthodontic devices including brackets and wires, implants, dentures, and orthodontic auxiliary tools inserted into the oral cavity). The three-dimensional scanner 200 may emit light to the oral cavity (e.g., at least one tooth or a gum of the target object 20) of the target object 20 by using a light source (or projector), and receive light reflected from the oral cavity of the target object 20, via a camera (or at least one image sensor). According to another embodiment, the three-dimensional scanner 200 may scan a diagnostic model of the oral cavity to obtain an image of the diagnostic model of the oral cavity. If the diagnostic model of the oral cavity is a diagnostic model obtained by taking an impression of the shape of the oral cavity of the target object 20, the image of the diagnostic model of the oral cavity may be an image of the oral cavity of the target object. Hereinafter, for convenience of explanation, a description is given under the assumption of a case where an image of the oral cavity of the target object 20 is obtained by scanning the inside of the oral cavity, but the disclosure is not limited thereto.

The three-dimensional scanner 200 according to various embodiments may obtain, as a two-dimensional image, a surface image of the oral cavity of the target object 20 based on information received via a camera. The surface image of the oral cavity of the target object 20 may include at least one of at least one tooth, a gum, an artificial structure, a cheek, the tongue, or a lip of the target object 20. The surface image of the oral cavity of the target object 20 may be a two-dimensional image.

A two-dimensional image of the oral cavity obtained in the three-dimensional scanner 200 according to various embodiments may be transmitted to an electronic device 100 connected thereto over a wired or wireless communication network. The electronic device 100 may be a computer device or a portable communication device. The electronic device 100 may generate a three-dimensional image (or a three-dimensional oral image or a three-dimensional oral model) of the oral cavity which three-dimensionally represents the oral cavity based on a two-dimensional image of the oral cavity received from the three-dimensional scanner 200. The electronic device 100 may generate a three-dimensional image of the oral cavity by three-dimensionally modeling an internal structure of the oral cavity based on a received two-dimensional image of the oral cavity.

The three-dimensional scanner 200 according to another embodiment may scan the oral cavity of the target object 20 to obtain a two-dimensional image of the oral cavity, generate a three-dimensional image of the oral cavity based on the obtained two-dimensional image of the oral cavity, and transmit the generated three-dimensional image of the oral cavity to the electronic device 100.

The electronic device 100 according to various embodiments may be communicatively connected to a cloud server (not illustrated). In the above case, the electronic device 100 may transmit a two-dimensional image of the oral cavity of the target object 20 or a three-dimensional image of the oral cavity to the cloud server, and the cloud server may store the two-dimensional image of the oral cavity of the target object 20 or the three-dimensional image of the oral cavity which is received from the electronic device 100.

According to another embodiment, as the three-dimensional scanner, a table scanner (not illustrated) fixed and used at a particular position may be used in addition a handheld scanner that is inserted into and used in the oral cavity of the target object 20. The table scanner may scan a diagnostic model of the oral cavity to generate a three-dimensional image of the diagnostic model of the oral cavity. In the above case, the diagnostic model of the oral cavity may be scanned by moving at least one of a light source (or projector) of the table scanner, a camera, or a jig to which the diagnostic model is fixed.

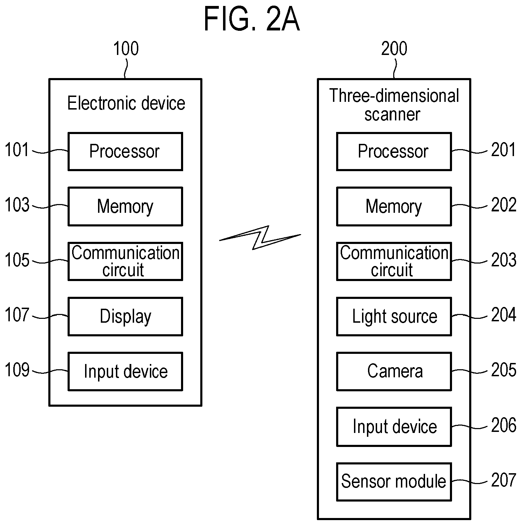

FIG. 2A is a block diagram of the electronic device 100 and the three-dimensional scanner 200 according to various embodiments of the present disclosure. The electronic device 100 and the three-dimensional scanner 200 may be communicatively connected to each other over a wired or wireless communication network, and transmit or receive various data to/from each other.

The three-dimensional scanner 200 according to various embodiments may include a processor 201, a memory 202, a communication circuit 203, a light source 204, a camera 205, an input device 206, and/or a sensor module 207. At least one of elements included in the three-dimensional scanner 200 may be omitted or other elements may be added to the three-dimensional scanner 200. Additionally or alternatively, some elements may be implemented integrally or may be implemented as a single or multiple entities. At least some elements in the three-dimensional scanner 200 may be connected to each other via a bus, a general purpose input/output (GPIO), a serial peripheral interface (SPI), or a mobile industry processor interface (MIPI) and may exchange data and/or signals with each other.

The processor 201 of the three-dimensional scanner 200 according to various embodiments may be an element capable of performing calculation or data processing related to control and/or communication of each element of the three-dimensional scanner 200, and may be operatively connected to elements of the three-dimensional scanner 200. The processor 201 may load, on the memory 202, a command or data received from another element of the three-dimensional scanner 200, process the command or data stored in the memory 202, and store result data. The memory 202 of the three-dimensional scanner 200 according to various embodiments may store instructions for operations of the processor 201 described above.

According to various embodiments, the communication circuit 203 of the three-dimensional scanner 200 may establish a wired or wireless communication channel with an external device (e.g., the electronic device 100) and transmit or receive various data to/from the external device. According to an embodiment, the communication circuit 203 may include at least one port for being connected to an external device through a wired cable, so as to perform wired communication with the external device. In the above case, the communication circuit 203 may communicate with an external device communicated by wire through the at least one port. According to an embodiment, the communication circuit 203 may include a cellular communication module and be configured to be connected to a cellular network (e.g., 3G, LTE, 5G, Wibro, or WiMAX). According to various embodiments, the communication circuit 203 may include a short-range communication module and perform data transmission or reception with an external device by using short-range communication (e.g., Wi-Fi, Bluetooth, Bluetooth Low Energy (BLE), or UWB), but the disclosure is not limited thereto. According to an embodiment, the communication circuit 203 may include a non-contact communication module for non-contact communication. The non-contact communication may include a proximity communication technology employing at least one non-contact scheme, such as near field communication (NFC), radio frequency identification (RFID) communication, or magnetic secure transmission (MST) communication.

The light source 204 of the three-dimensional scanner 200 according to various embodiments may emit light toward the oral cavity of the target object 20. For example, the light emitted from the light source 204 may be structured light having a predetermined pattern (e.g., a stripe pattern in which straight lines having different colors consecutively appear). The pattern of the structured light may be generated using a pattern mask or a digital micro-mirror device (DMD), but the disclosure is not limited thereto. The camera 205 of the three-dimensional scanner 200 according to various embodiments may obtain an image of the oral cavity of the target object 20 by receiving reflective light reflected by the oral cavity of the target object 20. The camera 205 may include a left camera corresponding to the sight of the left eye and a right camera corresponding to the sight of the right eye so as to construct a three-dimensional image according to, for example, optical triangulation. The camera 205 may include at least one image sensor, such as a CCD sensor or a CMOS sensor.

The input device 206 of the three-dimensional scanner 200 according to various embodiments may receive a user input for controlling the three-dimensional scanner 200. The input device 206 may include a button that receives a push input of the user 10, a touch panel that detects a touch of the user 10, and a voice recognition device including a microphone. For example, the user 10 may control to start or stop scanning by using the input device 206.

The sensor module 207 of the three-dimensional scanner 200 according to various embodiments may detect an operational state of the three-dimensional scanner 200 or an external environmental state (e.g., the user's operation), and generate an electrical signal corresponding to the detected state. The sensor module 207 may include, for example, at least one of a gyro sensor, an acceleration sensor, a gesture sensor, a proximity sensor, or an infrared sensor. The user 10 may control to start or stop scanning by using the sensor module 207. For example, in a case where the user 10 is moving while holding the three-dimensional scanner 200 with a hand, when an angular velocity measured by the sensor module 207 exceeds a predetermined threshold, the three-dimensional scanner 200 may control the processor 201 to start a scanning operation.

According to an embodiment, the three-dimensional scanner 200 may receive a user input for starting scanning via the input device 206 of the three-dimensional scanner 200 or the input device 206 of the electronic device 100, or may start scanning according to processing in the processor 201 of the three-dimensional scanner 200 or the processor 201 of the electronic device 100. When the user 10 scans the inside of the oral cavity of the target object 20 by means of the three-dimensional scanner 200, the three-dimensional scanner 200 may generate a two-dimensional image of the oral cavity of the target object 20, and transmit the two-dimensional image of the oral cavity of the target object 20 to the electronic device 100 in real time. The electronic device 100 may display the received two-dimensional image of the oral cavity of the target object 20 through a display. In addition, the electronic device 100 may generate (construct) a three-dimensional image of the oral cavity of the target object 20 based on a two-dimensional image of the oral cavity of the target object 20, and display the three-dimensional image of the oral cavity through the display. The electronic device 100 may display the three-dimensional image being generated, through the display in real time.

The electronic device 100 according to various embodiments may include one or more processors 101, one or more memories 103, a communication circuit 105, a display 107, and/or an input device 109. At least one of the elements included in the electronic device 100 may be omitted or other elements may be added to the electronic device 100. Additionally or alternatively, some elements may be implemented integrally or may be implemented as a single or multiple entities. At least some elements in the electronic device 100 may be connected to each other via a bus, a general purpose input/output (GPIO), a serial peripheral interface (SPI), or a mobile industry processor interface (MIPI) and may exchange data and/or signals with each other.

According to various embodiments, the one or more processors 101 of the electronic device may be elements capable of performing calculation or data processing related to control and/or communication of each element (e.g., the memory 103) of the electronic device 100. The one or more processors 101 may be operatively connected to, for example, elements of the electronic device 100. The one or more processors 101 may load, on the one or more memories 103, a command or data received from another element of the electronic device 100, process the command or data stored in the one or more memories 103, and store result data.

According to various embodiments, the one or more memories 103 of the electronic device 100 may store instructions for operations of the one or more processors 101. The one or more memories 103 may store correlation models constructed according to a machine learning algorithm. The one or more memories 103 may store data (e.g., a two-dimensional image of the oral cavity obtained through oral scanning) received from the three-dimensional scanner 200.

According to various embodiments, the communication circuit 105 of the electronic device 100 may establish a wired or wireless communication channel with an external device (e.g., the three-dimensional scanner 200 or the cloud server) and transmit or receive various data to/from the external device. According to an embodiment, the communication circuit 105 may include at least one port for being connected to an external device through a wired cable, so as to perform wired communication with the external device. In the above case, the communication circuit 105 may communicate with an external device communicated by wire through the at least one port. According to an embodiment, the communication circuit 105 may include a cellular communication module and be configured to be connected to a cellular network (e.g., 3G, LTE, 5G, Wibro, or WiMAX). According to various embodiments, the communication circuit 105 may include a short-range communication module and perform data transmission or reception with an external device by using short-range communication (e.g., Wi-Fi, Bluetooth, Bluetooth Low Energy (BLE), or UWB), but the disclosure is not limited thereto. According to an embodiment, the communication circuit 105 may include a non-contact communication module for non-contact communication. The non-contact communication may include a proximity communication technology employing at least one non-contact scheme, such as near field communication (NFC), radio frequency identification (RFID) communication, or magnetic secure transmission (MST) communication.

The display 107 of the electronic device 100 according to various embodiments may display various screens based on a control of the processor 101. The processor 101 may display, through the display 107, a two-dimensional image of the oral cavity of the target object 20 received from the three-dimensional scanner 200, and/or a three-dimensional image of the oral cavity obtained by three-dimensionally modeling an internal structure of the oral cavity. For example, the processor may display a two-dimensional image and/or a three-dimensional image of the oral cavity by means of a particular application program. In the above case, the user 10 may edit, store, and remove the two-dimensional image and/or the three-dimensional image of the oral cavity.

The input device 109 of the electronic device 100 according to various embodiments may receive a command or data to be used in an element (e.g., the one or more processors 101) of the electronic device 100 from the outside (e.g., from the user) of the electronic device 100. The input device 109 may include, for example, a microphone, a mouse or a keyboard. According to an embodiment, the input device 109 may be implemented in a type of a touch sensor panel that is combined with the display 107 to be able to recognize a contact or approach of various external objects.

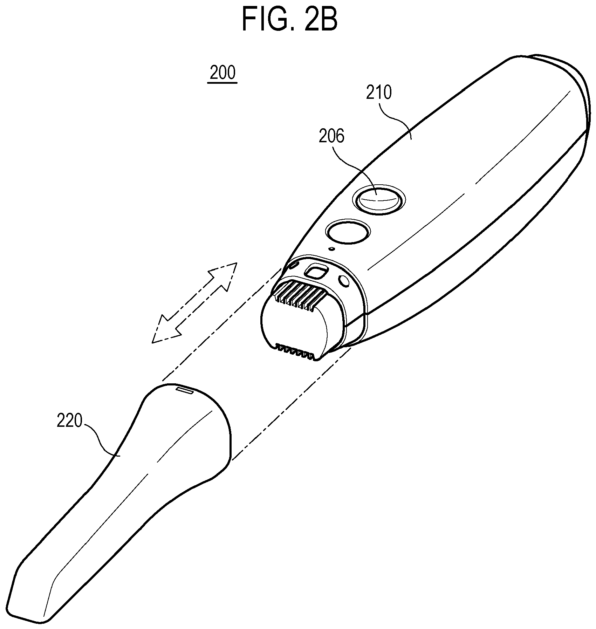

FIG. 2B is a perspective view of the three-dimensional scanner 200 according to various embodiments. The three-dimensional scanner 200 according to various embodiments may include a body 210 and a probe tip 220. The body 210 of the three-dimensional scanner 200 may have a shape that is easy to be gripped and used by the user 10 with a hand. The probe tip 220 may have a shape that is easy to be introduced into and discharged from the oral cavity of the target object 20. In addition, the body 210 may be coupled to and separated from the probe tip 220. In the body 210, the elements of the three-dimensional scanner 200 described with reference to FIG. 2A may be arranged. One end of one side of the body 210 may have an opening that is open to enable the light output from the light source 204 to be emitted to the target object 20. The light emitted through the opening may enter through the opening again after being reflected by the target object 20. The reflected light entering through the opening may be captured by the camera to generate an image of the target object 20. The user 10 may start scanning by using the input device 206 (e.g., button) of the three-dimensional scanner 200. For example, when the user 10 touches or presses the input device 206, the light from the light source 204 may be emitted to the target object 20.

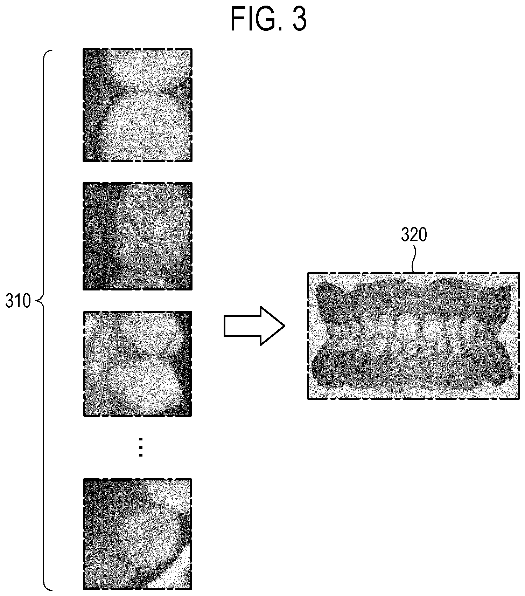

FIG. 3 is a diagram illustrating a method of generating a three-dimensional image 320 of an oral cavity according to various embodiments. The user 10 may scan the inside of the oral cavity of the target object 20 while moving the three-dimensional scanner 200, and in this case, the three-dimensional scanner 200 may obtain multiple two-dimensional images 310 of the oral cavity of the target object 20. For example, the three-dimensional scanner 200 may obtain a two-dimensional image of an area including an incisor of the target object 20 and a two-dimensional image of an area including a molar of the target object 20. The three-dimensional scanner 200 may transmit the obtained multiple two-dimensional images 310 to the electronic device 100. According to another embodiment, the user 10 may scan a diagnostic model of the oral cavity or obtain multiple two-dimensional images of the diagnostic model of the oral cavity while moving the three-dimensional scanner 200. Hereinafter, for convenience of explanation, a description is given under the assumption of a case where an image of the oral cavity of the target object 20 is obtained by scanning the inside of the oral cavity of the target object 20, but the disclosure is not limited thereto.

The electronic device 100 according to various embodiments may convert each of the multiple two-dimensional images 310 of the oral cavity of the target object 20 into a set of multiple points having three-dimensional coordinate values. For example, the electronic device 100 may convert each of the multiple two-dimensional images 310 into a point cloud that is a set of data points having three-dimensional coordinate values. For example, a point cloud set including three-dimensional coordinate values based on the multiple two-dimensional images 310 may be stored as raw data about the oral cavity of the target object 20. The electronic device 100 may align point clouds, each of which is a set of data points having three-dimensional coordinate values, thereby completing an entire teeth model.

The electronic device 100 according to various embodiments may reconfigure (reconstruct) a three-dimensional image of the oral cavity. For example, the electronic device 100 may use a Poisson algorithm to merge a point cloud set stored as raw data so as to reconfigure multiple points and convert same into a closed three-dimensional surface, thereby reconfiguring the three-dimensional image 320 of the oral cavity of the target object 20.

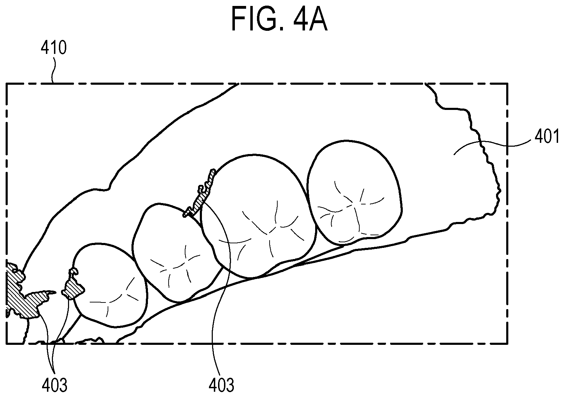

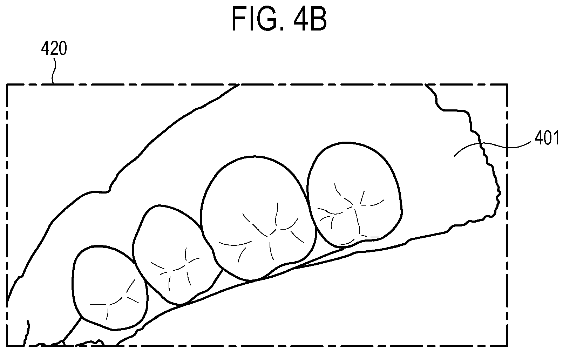

FIG. 4A and FIG. 4B are diagrams illustrating a process of performing noise filtering according to various embodiments of the present disclosure. FIG. 4A is a diagram illustrating a three-dimensional image model 410 of a target object including noise 403, and FIG. 4B is a diagram illustrating a three-dimensional image model 420 of the target object from which the noise has been removed through noise filtering disclosed herein.

The electronic device 100 according to various embodiments may obtain scan data values for the surface of the target object through a scan of the three-dimensional scanner 200, and may generate the three-dimensional image model 410 of the target object based on the obtained scan data values. The target object described herein may mean, for example, the oral cavity of a patient or a diagnostic model (e.g., a plaster model or an impression model) obtained by taking an impression of the shape of the oral cavity. The scan data values may include a three-dimensional coordinate value. The three-dimensional image model 410 of the target object may include the noise 403 irrelevant to teeth and a gum 401 of the target object according to various causes. Examples of causes for which the noise 403 occurs of FIG. 4A are as follows.

According to an embodiment, in relation to noise included in a three-dimensional image model of a target object, even when primary noise filtering is performed therefor, some noise may not be removed and remain. Specifically, if noise occurs in a three-dimensional image model, the electronic device 100 may scan the surface of a target object two times to perform a primary noise filtering operation. For example, when the target object is scanned at a first scan time point (first scan), if an obstacle (e.g., a finger) is scanned together, first scan data values obtained by the first scan include noise corresponding to the obstacle. In order to remove the noise, when the obstacle has disappeared, the target object may be scanned again (second scan) to obtain second scan data. Thereafter, vectors connecting the first scan data values to a virtual focal point of the three-dimensional scanner 200 are determined, whether the vectors pass through the second scan data values is determined, and when the vectors pass through the second scan data values, a data value, among the first scan data values, which is associated with at least one vector passing through a second scan data value is removed, whereby primary noise filtering may be performed. In this case, some noise may not be removed through the primary noise filtering and may still remain in the three-dimensional image model. Specifically, in the noise filtering, only when vectors connecting a virtual focal point and first scan data values pass through a second scan data value, only a scan data value, among the first scan data values, meeting a corresponding vector is considered as noise and is removed, and thus scan data values not meeting the vector may not be removed and remain. The noise filtering disclosed herein may be used to remove such remaining scan data values.

According to an embodiment, when a target object is scanned using the three-dimensional scanner 200, a teeth area or a gum area may be identified and a three-dimensional image model corresponding to the identified areas may be generated. In this case, areas (e.g., soft tissue area and tongue area) other than the teeth area or the gum area may be included as noise in the three-dimensional image model. Specifically, the electronic device 100 may perform machine learning of images in which a teeth area, a gum area, and other areas are labeled respectively, according to a machine learning algorithm so as to identify the teeth area or the gum area in an image of a target object. For example, a correlation between a two-dimensional image set of the oral cavities of target objects and a data set in which a teeth area and a gum area are identified in each image of the two-dimensional image set may be modeled according to a machine learning algorithm to construct a correlation model. The electronic device 100 may use the constructed correlation model to identify a teeth area or a gum area in multiple two-dimensional images of a target object, and generate a three-dimensional image model corresponding to the identified teeth area or gum area. In this case, a filtering operation for removing an area remaining after excluding the identified teeth area or gum area may be performed. Even when the filtering operation is performed, the remaining area may not be completely removed, and remain. For example, when a tongue area to be filtered out is misidentified as a gum area which is not to be filtered out, the area may not be removed by the filtering operation and may remain. The noise filtering disclosed herein may be used to remove such a remaining area.

According to an embodiment, when a target object is scanned using the three-dimensional scanner 200, external light (e.g., natural light) is reflected by a particular material (e.g., artificial structure) included in the target object, whereby noise may occur. For example, if metal, such as gold or amalgam, is included in a target object, the three-dimensional scanner 200 may receive external light reflected by the metal, and the light may cause noise in some areas of a three-dimensional image model of the target object. The noise filtering disclosed herein may be used to remove such noise generated in some areas.

According to an embodiment, a user may edit (e.g., remove) a three-dimensional image model of a target object by means of the input device 109, and such an edit process may cause noise. A user may select an area that the user wants to remove from a generated three-dimensional image model, by means of the input device 109 (e.g., mouse). For example, the user may use the input device 109 to select the area that the user wants to remove, in various shapes such as polygons, lines, dots, etc. In this case, the electronic device 100 may separate the selected area from the remaining area (or main cluster), and the separated area may be determined as noise. For example, if a user wants to remove a particular area from a three-dimensional image model, the user may select the border of the particular area by means of the input device 109. In this case, the selected border of the particular area may be removed from the three-dimensional image model. Accordingly, the particular area may be separated as a separate cluster different from the remaining area. In this case, the cluster corresponding to the particular area separated from the main cluster may be determined as noise. The noise filtering disclosed herein may be used to remove such noise.

Embodiments in which the noise described above may occur are examples, and noise may occur in a generated three-dimensional image model by other various causes. The noise filtering technique described herein may be used to remove noise generated in a three-dimensional image model.

The electronic device 100 according to various embodiments may perform noise filtering to remove the noise 403 included in the three-dimensional image model 410 of the target object of FIG. 4A. A detailed noise filtering method will be described later. The electronic device 100 may perform noise filtering to generate the three-dimensional image model 420 from which the noise has been removed as illustrated in FIG. 4B.

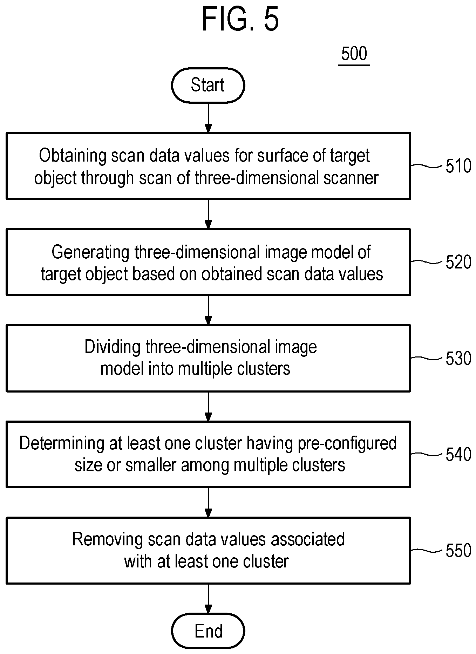

FIG. 5 is an operation flowchart of the electronic device 100 according to various embodiments of the present disclosure. Specifically, FIG. 5 is an operation flowchart illustrating a noise filtering method of the electronic device 100.

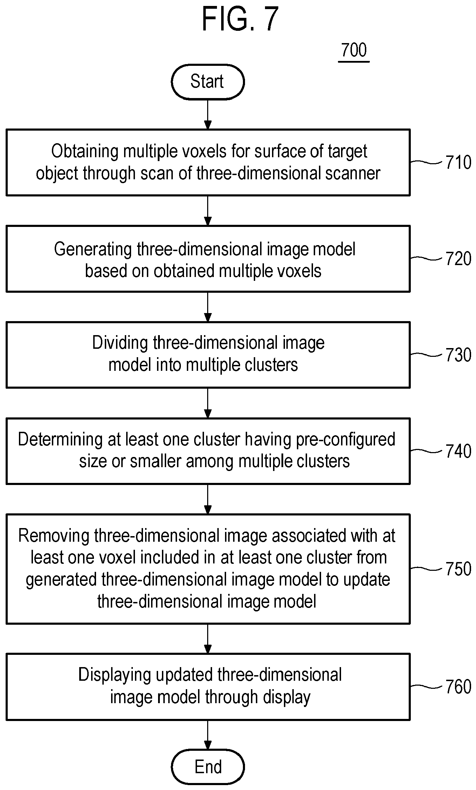

Referring to an operation flowchart 500, the electronic device 100 according to various embodiments may, in operation 510, obtain scan data values for the surface of a target object through a scan of the three-dimensional scanner 200. The scan data values may include a three-dimensional coordinate value. The three-dimensional coordinate value may be generated based on two-dimensional image data obtained by the three-dimensional scanner 200. The scan data values may include three-dimensional volume data represented by multiple voxels, and a case where a scan data value corresponds to a voxel will be described with reference to FIG. 7 later.

The electronic device 100 according to various embodiments may, in operation 520, generate a three-dimensional image model of the target object based on the obtained scan data values. The generated three-dimensional image model may be displayed on the display 107 of the electronic device 100. According to an embodiment, an alignment stage allowing generated three-dimensional volume data to be connected to each other and aligned may be additionally performed. The generated three-dimensional image model may include noise not intended by a user. In order to remove the noise, the electronic device 100 may perform noise filtering.

The electronic device 100 according to various embodiments may divide the three-dimensional image model into multiple clusters in operation 530. According to an embodiment, the electronic device 100 may divide the three-dimensional image model into multiple clusters through a method of determining, as one cluster, scan data values having consecutive three-dimensional coordinate values among the obtained scan data values. According to an embodiment, the electronic device 100 may determine, as multiple clusters, multiple closed curved surfaces included in the three-dimensional image model, thereby dividing the three-dimensional image model into the multiple clusters. The closed curved surface described above may mean a single surface defined by consecutive multiple three-dimensional coordinate values. For example, a closed curved surface included in the three-dimensional image model may be determined as one cluster.

The electronic device 100 according to various embodiments may determine at least one cluster having a size equal to or smaller than a predetermined size among the multiple clusters in operation 540. The determined at least one cluster may be considered as noise to be removed. The electronic device 100 may determine whether each of the multiple clusters corresponds to noise, based on the size of each of the multiple clusters.

According to an embodiment, the electronic device 100 may identify the number of voxels included in each of the multiple clusters, and determine, among the multiple clusters, at least one cluster having voxels, the number of which is equal to or smaller than a predetermined number. A method of determining a cluster to be removed based on the number of voxels will be described with reference to FIG. 7 later. Additionally, the electronic device 100 may determine a cluster to be finally removed among the determined at least one cluster based on a user input. The electronic device 100 may determine, among the multiple clusters, at least one cluster having voxels, the number of which is equal to or smaller than a predetermined number, and display the at least one cluster through the display 107 to be distinguished from other clusters. For example, the determined at least one cluster may be displayed using a color different from those of the other clusters. The user may directly select a cluster to be removed (or a cluster to be excluded from clusters to be removed) among the at least one cluster by means of the input device 109. The electronic device 100 may receive a user input for selecting a cluster to be removed (or a cluster to be excluded from clusters to be removed) among the at least one cluster, and determine a cluster to be finally removed based on the received user input.

According to an embodiment, the electronic device 100 may determine, as at least one cluster to be removed, clusters remaining after excluding a predetermined number of clusters from the multiple clusters in an order from the largest cluster size to the smallest. For example, the electronic device 100 may determine, as at least one cluster to be removed, clusters remaining after excluding a cluster having the largest size from the multiple clusters. For example, the electronic device 100 may determine, as at least one cluster to be removed, clusters remaining after excluding three clusters from the multiple clusters in an order from the largest cluster size to the smallest. The number of clusters remaining after noise filtering may be configured by the user's input.

According to an embodiment, the electronic device 100 may identify whether each of the multiple clusters corresponds to a teeth area or a gum area, and determine, among the multiple clusters, at least one cluster having a size equal to or smaller than a predetermined size and not corresponding to the teeth area or gum area. For example, when a target object is scanned using the three-dimensional scanner 200, the electronic device 100 may identify a teeth area and a gum area in multiple two-dimensional images of the target object, and mask the identified teeth area and gum area to be distinguished from other areas. The electronic device 100 may identify a teeth area and a gum area in a three-dimensional image model of the target object generated using the multiple two-dimensional images of the target object. The electronic device 100 may determine at least one cluster not corresponding to a teeth area or a gum area among clusters having a size equal to or smaller than a predetermined size among the multiple clusters. In the present embodiment, a teeth area or a gum area is masked, but this merely corresponds to an example, and a soft tissue area (e.g., a cheek area, a tongue area, or a lip area) or an artificial structure (e.g., orthodontic devices including brackets and wires, implants, dentures, orthodontic auxiliary tools inserted into the oral cavity, prosthesis, and abutments for supporting prosthesis) may also be masked.

The electronic device 100 according to various embodiments may remove scan data values associated with the at least one cluster. The electronic device 100 may remove scan data values associated with the at least one cluster and then update the generated three-dimensional image model. Through the above processes, the noise included in the three-dimensional image model can be effectively removed. The electronic device 100 may display the updated three-dimensional image model through the display 107.

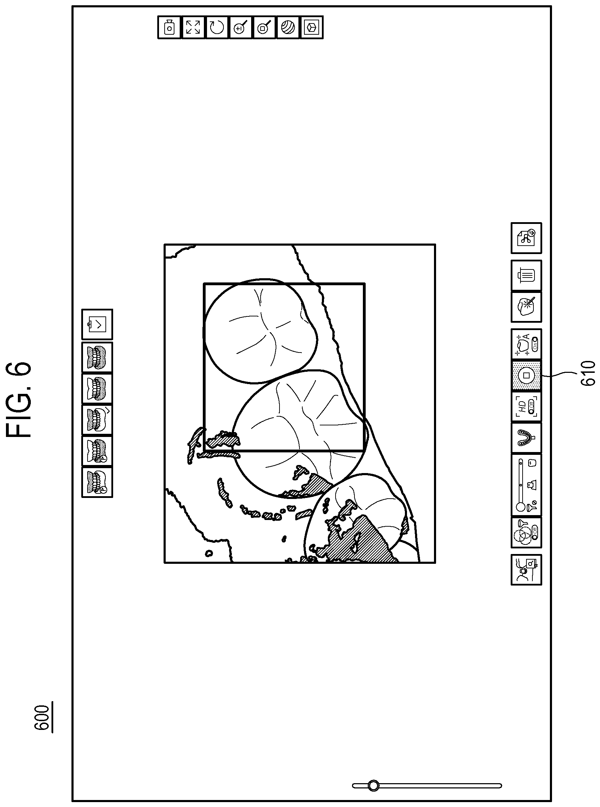

FIG. 6 illustrates an interface 600 for generating a three-dimensional image model of a target object according to various embodiments of the present disclosure.

According to various embodiments, when a user scans a target object by means of the three-dimensional scanner 200, the electronic device 100 may receive images of the target object from the three-dimensional scanner 200 in real time, generate (construct) a three-dimensional image model of the target object based on the received images, and display the three-dimensional image model of the target object through the display 107. The electronic device 100 may display a three-dimensional image model which is being generated as illustrated in FIG. 6 through the display 107 in real time.

The electronic device 100 according to various embodiments may receive a user input for terminating a scan of the three-dimensional scanner 200, through the input device 109. For example, the user may select a scan termination icon 610 displayed in the interface, by means of the input device 109. The electronic device 100 may perform a noise filtering operation in response to reception of a user input for terminating a scan of the three-dimensional scanner 200. For example, the electronic device 101 may, in response to reception of a user input for terminating a scan of the three-dimensional scanner 200, divide the three-dimensional image model into multiple clusters, determine at least one cluster having a size equal to or smaller than a predetermined size among the multiple clusters, and remove scan data values associated with the determined at least one cluster. In this case, the electronic device 100 may generate a three-dimensional image model of the target object from which noise has been removed, as illustrated in FIG. 4B.

FIG. 7 is an operation flowchart of the electronic device 100 according to various embodiments of the present disclosure. A description overlapping with the description given with reference to FIG. 5 is omitted. Referring to an operation flowchart 700, the electronic device 100 according to various embodiments may, in operation 710, obtain multiple voxels for the surface of a target object through a scan of the three-dimensional scanner 200. A voxel is graphic information defining one point in a three-dimensional space, and may include a three-dimensional coordinate value.

The electronic device 100 according to various embodiments may, in operation 720, generate a three-dimensional image model of the target object based on the obtained multiple voxels. According to an embodiment, an alignment stage allowing the generated voxels to be connected to each other and aligned may be additionally performed. The generated three-dimensional image model may include noise not intended by a user. In order to remove the noise, the electronic device 100 may perform noise filtering.

The electronic device 100 according to various embodiments may divide the three-dimensional image model into multiple clusters in operation 730. The electronic device 100 according to various embodiments may determine at least one cluster having a size equal to or smaller than a predetermined size among the multiple clusters in operation 740. The electronic device 100 may identify the number of voxels included in each of the multiple clusters, and determine, among the multiple clusters, at least one cluster having voxels, the number of which is equal to or smaller than a predetermined number. In this case, the electronic device 100 may identify how many voxels each cluster has. The electronic device 100 may determine, as at least one cluster to be considered as noise, a cluster having voxels, the number of which is equal to or smaller than a predetermined number, based on the number of voxels included in each of the multiple clusters.

The electronic device 100 according to various embodiments may, in operation 750, remove a three-dimensional image associated with at least one voxel included in the determined at least one cluster from the generated three-dimensional image model to update the generated three-dimensional image model. For example, the electronic device 100 according to various embodiments may remove a three-dimensional image associated with at least one voxel included in at least one cluster from the generated three-dimensional image model to update the three-dimensional image model. The electronic device 100 according to various embodiments may display the updated three-dimensional image model through the display 107 in operation 760.

Various embodiments of the present disclosure may be implemented as software recorded in a machine-readable recording medium. The software may be software for implementing the above-mentioned various embodiments of the present disclosure. The software may be inferred from various embodiments of the present disclosure by programmers in a technical field to which the present disclosure belongs. For example, the software may be a machine-readable command (e.g., code or a code segment) or program. A machine may be a device capable of operating according to an instruction called from the recording medium, and may be, for example, a computer. In an embodiment, the machine may be the device 100 according to embodiments of the present disclosure. In an embodiment, a processor of the machine may execute a called command to cause elements of the machine to perform a function corresponding to the command. In an embodiment, the processor may be the at least one processor 101 according to embodiments of the present disclosure. The recording medium may refer to any type of recording medium which stores data capable of being read by the machine. The recording medium may include, for example, a ROM, a RAM, a CD-ROM, a magnetic tape, a floppy disk, an optical data storage device, and the like. In an embodiment, the recording medium may be the at least one memory 103. In an embodiment, the recording medium may be distributed to computer systems which are connected to each other through a network. The software may be distributed, stored, and executed in the computer systems. The recording medium may be a non-transitory recording medium. The non-transitory recording medium refers to a tangible medium that exists irrespective of whether data is stored semi-permanently or temporarily, and does not include a transitorily transmitted signal.

Although the technical idea of the present disclosure has been described by the examples described in some embodiments and illustrated in the accompanying drawings, it should be noted that various substitutions, modifications, and changes can be made without departing from the technical scope of the present disclosure which can be understood by those skilled in the art to which the present disclosure pertains. In addition, it should be noted that such substitutions, modifications, and changes are intended to fall within the scope of the appended claims.

## Claims

1. An electronic device comprising:
a communication circuit communicatively connected to a three-dimensional scanner;
a display; and
one or more processors,
wherein the one or more processors are configured to:
obtain scan data values for a surface of a target object through a scan of the three-dimensional scanner, the scan data values including a three-dimensional coordinate value;
generate a three-dimensional image model of the target object, based on the obtained scan data values;
divide the three-dimensional image model into multiple clusters;
determine at least one cluster having a size equal to or smaller than a predetermined size among the multiple clusters; and
remove scan data values associated with the at least one cluster.

2. The electronic device of claim 1, wherein the one or more processors are configured to, after removing the scan data values, update the generated three-dimensional image model.

3. The electronic device of claim 2, wherein the one or more processors are configured to display the updated three-dimensional image model through the display.

4. The electronic device of claim 2, wherein the scan data values comprise multiple voxels, and
wherein the one or more processors are configured to remove a three-dimensional image associated with at least one voxel included in the at least one cluster from the generated three-dimensional image model, so as to update the three-dimensional image model.

5. The electronic device of claim 1, wherein the scan data values comprise multiple voxels, and
wherein the one or more processors are configured to determine the at least one cluster having voxels, the number of which is equal to or smaller than a predetermined number, among the multiple clusters.

6. The electronic device of claim 1, wherein the one or more processors are configured to determine, as the at least one cluster, a cluster remaining after excluding a predetermined number of clusters from the multiple clusters in an order from a largest cluster size to a smallest.

7. The electronic device of claim 1, further comprising an input device,
wherein the one or more processors are configured to divide the three-dimensional image model into the multiple clusters in response to reception of, through the input device, a user input for terminating the scan of the three-dimensional scanner.

8. The electronic device of claim 1, wherein the one or more processors are configured to determine, as one cluster, scan data values having consecutive three-dimensional coordinate values among the obtained scan data values.

9. The electronic device of claim 1, wherein the one or more processors are configured to determine, as the multiple clusters, multiple closed curved surfaces included in the three-dimensional image model.

10. The electronic device of claim 1, wherein the one or more processors are configured to:
determine whether each of the multiple clusters corresponds to a teeth area or a gum area; and
determine, among the multiple clusters, at least one cluster having a size equal to or smaller than a predetermined size and not corresponding to the teeth area or the gum area.

11. A method of processing a scan image of a three-dimensional scanner performed in an electronic device, the method comprising:
obtaining scan data values for a surface of a target object through a scan of the three-dimensional scanner, the scan data values including a three-dimensional coordinate value;
generating a three-dimensional image model of the target object, based on the obtained scan data values;
dividing the three-dimensional image model into multiple clusters;
determining at least one cluster having a size equal to or smaller than a predetermined size among the multiple clusters; and
removing scan data values associated with the at least one cluster.

12. The method of claim 11, further comprising, after the removing, updating the generated three-dimensional image model.

13. The method of claim 12, further comprising displaying the updated three-dimensional image model through the display.

14. The method of claim 12, wherein the scan data values comprise multiple voxels, and
wherein the updating comprises removing a three-dimensional image associated with at least one voxel included in the at least one cluster from the generated three-dimensional image model, so as to update the three-dimensional image model.

15. The method of claim 11, wherein the scan data values comprise multiple voxels, and
wherein the determining of the at least one cluster comprises determining the at least one cluster having voxels, the number of which is equal to or smaller than a predetermined number, among the multiple clusters.

16. The method of claim 11, wherein the determining the at least one cluster comprises determining, as the at least one cluster, a cluster remaining after excluding a predetermined number of clusters from the multiple clusters in an order from a largest cluster size to a smallest.

17. The method of claim 11, wherein the dividing of the three-dimensional image model into the multiple clusters comprises dividing the three-dimensional image model into the multiple clusters in response to reception of, through an input device, a user input for terminating the scan of the three-dimensional scanner.

18. The method of claim 11, wherein the dividing the three-dimensional image model into the multiple clusters comprises determining, as one cluster, scan data values having consecutive three-dimensional coordinate values among the obtained scan data values.

19. The method of claim 11, wherein the dividing the three-dimensional image model into the multiple clusters comprises determining, as the multiple clusters, multiple closed curved surfaces included in the three-dimensional image model.

20. The method of claim 11, wherein the determining the at least one cluster comprises:
determining whether each of the multiple clusters corresponds to a teeth area or a gum area; and
determining, among the multiple clusters, at least one cluster having a size equal to or smaller than a predetermined size and not corresponding to the teeth area or the gum area.
